# EUROPÄISCHE PATENTSCHRIFT

(11) **EP 1 102 635 B1**
(45) Veröffentlichungstag und Bekanntmachung des Hinweises auf die Patenterteilung: **09.10.2002**
(21) Anmeldenummer: 99940035.1
(22) Anmeldetag: 22.07.1999
(51) Int. Cl.: B01J 37/02, B01J 23/58, B01J 23/66, C07C 67/055, B01J 23/52

(54) **VERFAHREN ZUR HERSTELLUNG VON TRÄGERKATALYSATOREN SOWIE DEREN VERWENDUNG FÜR DIE HERSTELLUNG VON VINYLACETATMONOMER**
METHOD FOR PRODUCING SUPPORTED CATALYSTS AND THEIR USE FOR PRODUCING VINYL ACETATE MONOMER
PROCEDE POUR LA PRODUCTION DE CATALYSEURS SUPPORTES, AINSI QUE LEUR UTILISATION POUR LA PRODUCTION DE MONOMERE D'ACETATE VINYLIQUE

(30) Priorität: 31.07.1998 DE 19834569
(43) Veröffentlichungstag der Anmeldung: 30.05.2001
(73) Patentinhaber: Celanese Chemicals Europe GmbH, 60439 Frankfurt am Main (DE)
(72) Erfinder: HAGEMEYER, Alfred, Sunnyvale, CA 94086 (US); WERNER, Harald, D-61350 Bad Homburg (DE); DINGERDISSEN, Uwe, D-64342 Seeheim-Jugenheim (DE)
(86) Internationale Anmeldenummer: EP9905240
(87) Internationale Veröffentlichungsnummer: WO00007727

(56) Entgegenhaltungen:
- EP-A- 0 431 478
- EP-A- 0 519 435
- EP-A- 0 685 451
- EP-A- 0 723 810

## Beschreibung

Die vorliegende Erfindung beschreibt einen mit Hf dotierten Palladiumhaltigen Trägerkatalysator, ein Verfahren zu seiner Herstellung und seine Verwendung für die Vinylacetatsynthese.

Es ist bekannt, Vinylacetat (VAM) in der Gasphase aus Ethylen, Essigsäure und Sauerstoff herzustellen; die für diese Synthese verwendeten Trägerkatalysatoren enthalten Pd als Aktivmetall und ein Alkalielement als Promotor, vorzugsweise K. Als weitere Zusätze werden Cd, Au oder Ba verwendet. Die Metallsalze können durch Tränken, Imprägnieren, Aufsprühen, Aufdampfen, Tauchen oder Ausfällen auf den Träger aufgebracht werden.

US-A-3 743 607 und GB-1 333 449 beschreiben die Herstellung von Pd/Au-Trägerkatalysatoren für die VAM-Synthese durch Tränkung mit Pd/Au-Salzen und anschließende Reduktion. Mit dieser Methode entstehen aber keine Schalenkatalysatoren, sondern die Edelmetalle sind über den gesamten Pelletquerschnitt gleichmäßig verteilt.

GB- 1 283 737 offenbart die Herstellung eines Edelmetall-Schalenkatalysators durch Vortränkung des Trägers mit einer alkalischen Lösung und Sättigung mit 25-90% Wasser oder Alkohol. Die anschließende Imprägnierung mit Pd-Salzen und die Reduktion der abgeschiedenen Salze zum Metall ergibt Schalenkatalysatoren, wobei die Eindringtiefe der Edelmetalle bis zu 50% des Pelletradius betragen soll.

Bei den Pd/Au/K-Katalysatoren hat es sich als vorteilhaft erwiesen, die beiden Edelmetalle in Form einer Schale auf den Träger aufzubringen, d.h. die Edelmetalle sind nur in einer oberflächennahen Zone verteilt, während die weiter innen liegenden Bereiche des Trägerformkörpers nahezu edelmetallfrei sind. Die Schichtdicke dieser katalytisch aktiven Schalen beträgt ca. 0.1 - 2 mm.

Gemäß US-A-3 775 342 und US-A-3 822 308 werden Schalenkatalysatoren erzeugt durch Tränkung des Trägers mit einer Lösung aus Pd/Au-Salzen und mit einer wässrigen Base, vorzugsweise NaOH, wobei unlösliche Pd- und Au-Hydroxide in einer schalenförmigen Oberflächenzone auf den Pellets ausfallen. Die auf diese Weise in der Schale fixierten Hydroxide werden dann zu den Metallen reduziert.

GB 1 521 652 erhält nach dergleichen Vorgehensweise (Vorimprägnierung mit Pd-,Au-Salzen, Trocknung, Basenfällung, Reduktion) Schalenkatalysatoren vom eggwhite-Typ, d.h. nur ein innerer Ring des kugelförmigen SiO₂-Trägers enthält die Edelmetalle, während der innere Kern und eine dünne äußere Schale nahezu edelmetallfrei bleiben.

Nach EP-A-0 723 810 wird durch Vorbehandlung (Tränkung) des Trägers mit Metallsalzlösungen ein bevorzugt mit Al, Zr, Ti dotierter Träger hergestellt, der anschließend für die oben beschriebene Basenfällung zur Ausbildung eines Pd/Au/K-Schalenkatalysators eingesetzt wird. Als Ausgangssubstanzen für die Dotierung werden insbesondere teure Alkoholate, z.B. Zr-Alkoholate, verwendet. Die erhaltenen Katalysatoren müßen abschließend bei erhöhten Temperaturen getrocknet und bei Temperaturen bis zu 800 °C calciniert werden.

Aufgabe der vorliegenden Erfindung ist es somit, ein einfaches und kostengünstiges Verfahren zur Herstellung von aktiven und selektivenVAM Katalysatoren, insbesondere Schalenkatalysatoren, mit hoher Standzeit auf Basis Pd herzustellen.

Die vorliegende Erfindung betrifft daher ein Verfahren zur Herstellung eines Trägerkatalysators für die Produktion von Vinylacetatmonomer durch Imprägnieren des Trägers mit einer basischen Lösung und einer Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliverbindungen , die sich unter den Reaktionsbedingungen bei der Produktion von Vinylacetatmonomer ganz oder teilweise in Alkaliacetate umwandeln, wobei der Träger gleichzeitig mit der Edelmetallimprägnierung oder in einer Nachbehandlung mit einer oder mehrerer Hafniumverbindungen imprägniert und bei einer Temperatur ≤ 160 °C getrocknet und anschließend nicht kalziniert wird.

Es wurde nun gefunden, daß eine Hf-Dotierung vorteilhaft ist für die Aktivität sowie die Selektivität und eine bessere Edelmetallhaftung auf dem Träger garantiert wird. Außerdem wurde gefunden, daß bei der Herstellung der erfindungsgemäßen Katalysatoren unter Verwendung von Hafnium als Aktivator der zusätzliche Calcinierungsschritt entfallen kann.

Die erfindungsgemäßen Hafnium-dotierten Katalysatoren weisen ferner eine einheitlichere Pd bzw. Pd/Au-Aktivmetall-Verteilung und eine höhere Edelmetall-Dispersion auf als undotierte VAM-Katalysatoren. Die hohe Dispersion bleibt auch im Dauerbetrieb aufgrund verminderter Agglomeration der Edelmetallteilchen weitgehend erhalten, wodurch die Deaktivierung der erfindungsgemäßen Katalysatoren verlangsamt wird und lange Standzeiten resultieren.

Erfindungsgemäß wird ein poröser Träger, vorzugsweise ein geformter SiO₂ Träger, mit Hf dotiert, indem der poröse Träger während oder nach der Beladung mit einem Pd-Precursor bzw. der Fixierung/Reduktion des Pd-Precursors zum Pd-Metall mit einem geeigneten Hf-Precursor beauschlagt wird.
Bevorzugt wird der Träger mit einer Palladium- oder Palladium- und Goldsalze und eine oder mehrere Hafniumverbindungen enthaltenden Lösung imprägniert.

In einer bevorzugten Ausführungsform der Erfindung wird die Hf-Dotierung durch Tränken aus wässriger oder essigsaurer oder alkoholischer Lösung mit einem löslichen Hf-Precursor vorgenommen. Bevorzugt ist ein wasserlöslicher Precursor. Zwischen der Hf-Beaufschlagung und der Beladung mit den anderen Elementen können weitere Vor- oder Nachbehandlungsschritte eingefügt werden wie z.B. Waschen, Trocknen, Oxidieren oder Reduzieren.

Als Trägermaterial können alle üblicherweise einsetzbaren Trägermaterialien verwendet werden, wie z.B. SiO₂ oder Al₂O₃, deren Oxidgemische und Mischoxide (Alumosilikate) oder auch C. Bevorzugt ist SiO₂.
Der SiO₂- Träger wird vorzugsweise als Formkörper eingesetzt und liegt in Form von Kugeln, Tabletten, Ringen, Sternen oder anderen technischen Formkörpem vor. Der Durchmesser bzw. die Länge und Dicke der Trägerteilchen liegt im allgemeinen bei 3 bis 9 mm. Die Oberfläche der Träger liegt, gemessen mit der BET (nach Brunnauer, Emmet und Teller)-Methode, im allgemeinen bei 10 - 500 m²/g, bevorzugt bei 20 - 250 m²/g. Das Porenvolumen liegt im allgemeinen bei 0,3 bis 1,2 ml/g.

Der Hafnium-Gehalt der erfindungsgemäßen Katalysatoren liegt im Bereich von 0,01 und 50 Gew.-%. Bevorzugt ist ein Hf-Gehalt im Bereich von 0,05 bis 25 Gew.-%

Als Hf-Precursoren für die SiO₂-Dotierung sind alle anorganischen Hf-Salze und metallorganischen Hf-Verbindungen geeignet, die löslich sind und keine für den Katalysator giftigen Bestandteile, wie z.B. Schwefel enthalten. Bevorzugte Precursoren sind Hf-Oxichlorid = HfOCl₂, Hf-Chlorid = HfCl₄, substituierte Hafnocen-Dichloride, substituierte Cyclopentadienyl-Hf-Trichloride, Hf-Acetylacetonat, Hf-Alkoxide wie Ethoxid oder Butoxid oder Propoxid, Hf-Oxid, Hf-Hydroxid, Hf-Nitrat, Hf-thd (= Hf(C₁₁H₁₉O₂)₄).
Durch weitere Nachbehandlungen des mit dem oder den Hf-Precursoren belegten Katalysators wie z.B. Wasch- und Trocknungsschritte, Calcinierschritte, die Fixierung und Reduktion der Pd-Aktivkomponente, die Nachtränkung mit Alkali-Acetat-Promotoren etc. kann der Hf-Precursor in die endgültige Verbindung wie z.B. das Oxid oder Oxichlorid überführt werden, die dann im gebrauchsfertigen Katalysator vorliegt.

Die Belegung mit der Aktivkomponente Pd und den weiteren Promotoren wie Au, Cd, Ba, K kann nach den bekannten Methoden des Standes der Technik erfolgen. Im wesentlichen haben sich für die Vinylacetatsynthese die Katalysatorsysteme auf Basis Pd/Cd/K, Pd/Ba/K oder Pd/Au/K durchgesetzt.

Die mit dem erfindungsgemäßen Verfahren hergestellten Katalysatoren weisen im allgemeinen folgende Metallgehalte auf:
Der Pd-Gehalt der Pd/K/Cd- und der Pd/K/Ba-Katalysatoren beträgt im allgemeinen 0,6 bis 3,5 Gew.-%, vorzugsweise 0,8 bis 3,0 Gew.-%, insbesondere 1,0 bis 2,5 Gew.-%. Der Pd-Gehalt der Pd/Au/K-Katalysatoren beträgt im allgemeinen 0,5 bis 2,0 Gew.-%, vorzugsweise 0,6 bis 1,5 Gew.-%.

Der K-Gehalt beträgt im allgemeinen 0,5 bis 4,0 Gew.-%, vorzugsweise 1,5 bis 3,0 Gew.-%.
Der Cd-Gehalt der Pd/K/Cd-Katalysatoren beträgt im allgemeinen 0,1 bis 2,5 Gew.-%, vorzugsweise 0,4 bis 2,0 Gew.-%.
Der Ba-Gehalt der Pd/K/Ba-Katalysatoren beträgt im allgemeinen 0,1 bis 2,0 Gew.-%, vorzugsweise 0,2 bis 1,0 Gew.-%.
Der Au-Gehalt der Pd/K/Au-Katalysatoren beträgt im allgemeinen 0,2 bis 1,0 Gew.-%, vorzugsweise 0,3 bis 0,8 Gew.-%.

In einer bevorzugten Ausführungsform der Erfindung, enthält der Katalysator die folgende Zusammensetzung:

PdₓAu_{y}Hf_{z}

wobei x= 0,7-1,3 Gew.%, insbesondere 0,9 bis 1,1,
y= 0 bis 1 Gew.%, vorzugsweise 0,5-1 Gew%, insbesondere 0,6 bis 0,9,
z= 0,05 bis 5 Gew.%, insbesondere 0,1 bis 2 .

Als Salze sind alle Salze von Palladium, Cadmium, Barium; Gold und Kalium geeignet, die löslich sind und keine für den Katalysator giftigen Bestandteile, wie z.B. Schwefel enthalten. Bevorzugt sind die Acetate und die Chloride. Im Falle der Chloride sind PdCl₂, NaPdCl₄ und HAuCl₄ besonders bevorzugte Precursoren. Dabei muß aber im Falle der Chloride sichergestellt werden, daß die Chloridionen vor dem Einsatz des Katalysators entfernt werden. Dies geschieht durch Auswaschen des dotierten Trägers, z.B. mit Wasser, nachdem Pd und gegebenenfalls Au durch Reduktion zu den Metallen auf dem Träger fixiert wurden.

Als Lösungsmittel sind alle Verbindungen geeignet, in denen die gewählten Salze löslich sind und die nach der Imprägnierung leicht wieder durch Trocknung zu entfernen sind. Geeignet sind für die Acetate vor allem unsubstituierte Carbonsäuren, insbesondere Essigsäure. Für die Chloride ist vor allem Wasser geeignet. Die zusätzliche Verwendung eines weiteren Lösungsmittels ist dann zweckmäßig, wenn die Salze in der Essigsäure oder im Wasser nicht genügend löslich sind. Als zusätzliche Lösungsmittel kommen diejenigen in Betracht, die inert und mit Essigsäure bzw. Wasser mischbar sind. Genannt seien als Zusätze für Essigsäure Ketone wie Aceton und Acetylaceton, ferner Ether wie Tetrahydrofuran oder Dioxan, Acetonitril, Dimethylformamid, aber auch Kohlenwasserstoffe wie Benzol.

Von jedem der auf die SiO₂-Trägerteilchen aufzubringenden Elemente (Pd/K/Au/Hf, Pd/K/Cd/Hf, Pd/K/Ba/Hf) muß mindestens ein Salz aufgebracht werden. Man kann mehrere Salze eines Elements aufbringen, aber im allgemeinen bringt man von jedem der drei Elemente genau ein Salz auf. Die notwendigen Salzmengen können in einem Schritt oder durch Mehrfachimprägnierung aufgebracht werden. Die Salze können nach bekannten Methoden wie Tränken, Imprägnieren, Aufsprühen, Aufdampfen, Tauchen oder Ausfällen auf den Träger aufgebracht werden.

Als Reduktionsmittel sind alle Verbindungen geeignet, die die eingesetzten Pd- und Au-Salze zu den Metallen zu reduzieren vermögen. Als Reduktionsmittel kommen daher z.B. die Citrate, Formiate, Hydrazin, Hydroxylamin und Alkaliborhydride infrage. Gasförmige Reduktionsmittel wie H₂ oder CO oder Ethylen können ebenfalls verwendet werden, bei Schalenkatalysatoren allerdings nur, wenn bei der Imprägnierung mit den Metallsalzen bereits eine Schalenstruktur erzeugt worden ist.

Die Herstellung des Vinylacetats erfolgt im allgemeinen durch Überleiten von Essigsäure, Ethylen und Sauerstoff oder Sauerstoff enthaltenden Gasen bei Temperaturen von 100 bis 220°C, vorzugsweise 120 bis 200°C, und bei Drucken von 1 bis 25 bar, vorzugsweise 1 bis 20 bar, über den fertigen Katalysator, wobei nicht umgesetzte Komponenten im Kreis geführt werden können. Zweckmäßig hält man die Sauerstoffkonzentration unter 10 Vol.-% (bezogen auf das essigsäurefreie Gasgemisch). Unter Umständen ist jedoch auch eine Verdünnung mit inerten Gasen wie Stickstoff oder Kohlendioxid vorteilhaft. Besonders Kohlendioxid eignet sich zur Verdünnung, da es in geringen Mengen während der Reaktion gebildet wird.

Die folgenden Beispiele sollen die Erfindung erläutern.

### Beispiel 1

### (1% Hf, nicht calciniert):

1,67 g Palladiumdichlorid PdCl₂, 1.40 g Goldsäure-Trihydrat HAuCl₄*3H₂O und 2,29 g Hafniumdichlorid-oxid-Oktahydrat HfOCl₂*8H₂O werden in 75 ml Wasser gelöst und zu 100 g SiO₂-Trägerpellets vom Typ KA-160 (Fa. Südchemie) gegeben (Porenfüllmethode). Nach Aufziehen der Lösung wird am Rotavapor unter Überleiten von Luft bei 70°C gleichmäßig eingetrocknet und bei 110°C im Trockenschrank über Nacht nachgetrocknet. Nun werden 2,70 g KOH in 75 ml Wasser gelöst und zu den getrockneten Pellets gegeben, für 30 Minuten am Rotavapor gut gemischt, über Nacht stehengelassen und dann mit ca. 10 I Wasser im Soxhlet chloridfrei gewaschen. Nach Stehen über Nacht wird bei 110°C im Trockenschrank getrocknet.

Der Kontakt wird in 10% Ethylen/90% N₂ für 2h bei 150°C reduziert.

Anschließend werden 2,6 g Kaliumacetat in 25 ml Wasser gelöst, zu den Pellets gegeben und gut gemischt. Es wird über Nacht bei 110°C getrocknet.

Der fertige Katalysator enthält 1% Pd, 0.7% Au und 1% Hf.

### Beispiel 2 (Vergleichsbeispiel)

### (1% Hf, calciniert 4h bei 300°C in Luft):

Der Kontakt wurde analog Beispiel 1 hergestellt, mit dem einzigen Unterschied, daß nach der KOH-Fällung und vor der Chlorid-Auswaschung für 4h bei 300°C in Luft calciniert wurde.

### Beispiel 3 (Vergleichsbeispiel)

### (1% Hf, calciniert 4h bei 400°C in Luft):

Der Kontakt wurde analog Beispiel 1 hergestellt, mit dem einzigen Unterschied, daß nach der KOH-Fällung und vor der Chlorid-Auswaschung für 4h bei 400°C in Luft calciniert wurde.

### Beispiel 4

### (5% Hf, nicht calciniert):

Der Kontakt wurde analog Beispiel 1 (HAWE65) hergestellt, mit dem Unterschied, daß 11.47 g HfOCl2*8H2O (anstelle von 2.29 g HfOCl2*8H2O) vorgelegt wurden. Der Kontakt wurde nicht calciniert.

Der fertige Katalysator enthält 1% Pd, 0.7% Au und 5% Hf.

### Beispiel 5 (Vergleichsbeispiel)

### (5% Hf, calciniert 4h bei 300°C in Luft):

Der Kontakt wurde analog Beispiel 4 hergestellt, mit dem einzigen Unterschied, daß nach der KOH-Fällung und vor der Chlorid-Auswaschung für 4h bei 300°C in Luft calciniert wurde.

### Beispiel 6 (Vergleichsbeispiel)

### (5% Hf, calciniert 4h bei 400°C in Luft):

Der Kontakt wurde analog Beispiel 4 hergestellt, mit dem einzigen Unterschied, daß nach der KOH-Fällung und vor der Chlorid-Auswaschung für 4h bei 400°C in Luft calciniert wurde.

### Beispiel 7 (Vergleichsbeispiel)

Beispiel 7 entspricht dem Beispiel 1 mit dem Unterschied, daß auf die Hf-Dotierung verzichtet wurde.

### Beispiel 8

### (2% Hf, Cl-haltig):

0,84g Palladiumchlorid, 0,70g Goldsäure und 2,29g Hafniumdichlorid-oxid werden zusammen in 35 ml Wasser gelöst und zu den Pellets gegeben, es wird bei 60°C an der Oelpumpe 3h getrocknet.
1,35g KOH werden in 35 ml Wasser gelöst und zu den getrockneten Pellets gegeben. Man läßt über Nacht stehen. Es wird mit ca. 8l Wasser gewaschen, über Nacht bei 110°C getrocknet, mit C2H4 bei 170°C reduziert. 4g Kaliumacetat werden in 40ml Wasser gelöst und zu den Pellets gegeben, es wird über Nacht bei 110°C getrocknet.

### Beispiel 9

### (2% Hf)

2,29g Hafniumdichlorid-oxid werden zunächst in 35 ml Wasser gelöst und zu den Pellets gegeben. Es wird an der Oelpumpe getrocknet. 1,06 g Palladiumacetat, 0,81g Goldacetat werden in 35 ml Eisessig gelöst und zu den Pellets gegeben. Man läßt w. o. eintrocknen. 1,35g KOH werden in 40ml Wasser gelöst und zu den Pellets gegeben. Man läßt über Nacht stehen. Es wird mit ca. 8l Wasser im Soxhlet gewaschen, nach dem Waschen über Nacht bei 110°C getrocknet, mit C₂H₄ bei 170°C 3h reduziert. 4g Kaliumacetat werden in 40ml Wasser gelöst und zu den Pellets gegeben. Man trocknet über Nacht bei 110°C im Trockenschrank.

### Beispiel 10

### (0.5% Hf)

1,06g Palladiumacetat und 0,81g Goldacetat werden in 40 ml Eisessig heiß gelöst, 0,58g Hafniumdichlorid-oxid zugegeben und die Lösung nach 2 Min zu den Pellets gegeben. Es wird bei 60°C an der Oelpumpe möglichst viel Eisessig abgezogen und über Nacht bei 60°C im Vakuumtrockenschrank getrocknet. 1,40g KOH werden in 40ml Wasser gelöst und zu den Pellets gegeben. Man läßt über Nacht stehen. Es wird mit ca. 6l Wasser im Soxhlet gewaschen, über Nacht im Vakuumtrockenschrank bei 110°C getrocknet und 2h bei 170°C in 10% C₂H₄ reduziert. 4g Kaliumacetat werden in 40ml Wasser gelöst und zu den Pellets gegeben. Es wird über Nacht bei 110°C getrocknet.

### Beispiel 11

### (0.25% Hf)

1,06g Palladiumacetat und 0,81g Goldacetat werden in 40 ml Eisessig heiß gelöst, 0,29g Hafniumdichlorid-oxid zugegeben und die Lösung nach 2 Min zu den Pellets gegeben. Es wird bei 60°C an der Oelpumpe möglichst viel Eisessig abgezogen. 1,40g KOH werden in 40ml Wasser gelöst und zu den Pellets gegeben. Man läßt über Nacht stehen. Es wird im Soxhlet mit ca. 8l Wasser gewaschen, über Nacht im Vakuumtrockenschrank bei 110°C getrocknet und 2h bei 170°C in 10% C₂H₄ reduziert. 4g Kaliumacetat werden in 40ml Wasser gelöst und zu den Pellets gegeben. Es wird über Nacht bei 110°C im Vakuum getrocknet.

Eine Übersicht über die hergestellten Hf-dotierten Katalysatoren gibt die nachfolgende Tabelle:

| Beispiel | Hf-Beladung | Calcinieren |
|---|---|---|
| 5 | 5 % | 4h 300°C in Luft |
| 6 | 5 % | 4h 400°C in Luft |
| 4 | 5 % | nicht calciniert |
| 2 | 1 % | 4h 300°C in Luft |
| 3 | 1 % | 4h 400°C in Luft |
| 1 | 1 % | nicht calciniert |
| 7 | 0 | nicht calciniert |
| 8 | 2 % | nicht calciniert |
| 9 | 2 % | nicht calciniert |
| 10 | 0.5 % | nicht calciniert |
| 11 | 0.25 % | nicht calciniert |

Reaktortests für die Gasphasenoxidation von Ethylen und Essigsäure zu Vinylacetat:
Die Katalysatoren werden in einem Festbett-Rohrreaktor mit 2 cm Rohrduchmesser getestet. Der Reaktor wird von außen mit einer Öl-Mantelheizung temperiert. Es werden 15 ml der Katalysator-Formkörper vorgelegt. Das Reaktorvolumen vor und nach der Katalysatorschüttung wird mit Glaskugeln aufgefüllt. Die Testapparatur wird von einem Prozeßleitsystem gesteuert und kontinuierlich betrieben.
Der Katalysator wird zunächst aktiviert und dann unter konstanten Reaktionsbedingungen getestet.
Die Aktivierung besteht aus mehreren Schritten: Aufheizen unter N₂, Zugabe von Ethylen, Druckerhöhung, Zugabe Essigsäure, Halten der Bedingungen, Zugabe Sauerstoff.
Die Reaktionsbedingungen bei den Tests sind 160-170°C Reaktionstemperatur, 8-9 bar Überdruck. Der Feed setzt sich zusammen aus 64.5 Vol.-% Ethylen, 16.1 Vol.-% N₂, 14.3 Vol.-% Essigsäure und 5.1 Vol.-% O₂. Eine Vollanalyse des Reaktoraustrags wird direkt am Reaktorausgang mittels on-line GC (2 Säulen-Schaltung) durchgeführt.

Aus den GC-Daten wurden die VAM-Selektivitäten S ( = Mole VAM/(Mole VAM + 0.5 * Mole COₓ) und RZA (Raum-Zeit-Ausbeute = g VAM / I Kat. * h) bestimmt:

| Beispiel Nr. | Hf-Beladung % | T (°C) | p (bar) | S % | RZA g/l*h |
|---|---|---|---|---|---|
| 1 | 1 | 160 | 9 | 79 | 700 |
| 1 | 1 | 165 | 9 | 78 | 740 |
| 1 | 1 | 170 | 9 | 77 | 750 |
| 2 | 1 | 170 | 9 | 70 | 150 |
| 4 | 5 | 170 | 9 | 84 | 690 |
| 5 | 5 | 170 | 9 | 96 | 320 |
| 7 | 0 | 170 | 9 | 88 | 850 |
| 8 | 2 | 170 | 9 | 93 | 330 |
| 9 | 2 | 170 | 9 | 88 | 670 |
| 10 | 0.5 | 170 | 9 | 89 | 1100 |
| 11 | 0.25 | 170 | 9 | 90 | 820 |

Wie aus der Tabelle zu ersehen ist zeichnen sich mit Hf dotierte und nicht calcinierte Kontakte durch eine erhöhte Leistung gegenüber dem undotierten Kontakt aus. Calcinierte Katalysatoren sind durchwegs schlecht. Die Calcinierung führt zum Sintem und damit zur Deaktivierung der Edelmetallteilchen auf dem Träger.

## Patentansprüche

1. Verfahren zur Herstellung eines Trägerkatalysators durch Imprägnieren des Trägers mit einer basischen Lösung und einer Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliverbindungen, die sich unter den Reaktionsbedingungen bei der Produktion von Vinylacetatmonomer ganz oder teilweise in Alkaliacetate umwandeln, wobei der Träger gleichzeitig mit der Edelmetallimprägnierung oder in einer Nachbehandlung mit einer oder mehrerer Hafniumverbindungen imprägniert und bei einer Temperatur ≤ 160°C getrocknet und anschließend nicht kalziniert wird.

2. Verfahren nach Anspruch 1, **dadurch gekennzeichnet, daß** der Träger mit einer Lösung imprägniert wird, die außer den Palladiumsalzen und der oder den Hafniumverbindungen noch Au, Cd und/oder Ba-Verbindungen als Aktivatoren enthält.

3. Verfahren nach Anspruch 1 oder 2, **dadurch gekennzeichnet, daß** in Wasser, Essigsäure oder Alkohol lösliche Hafniumverbindungen verwendet werden.

4. Verfahren nach Anspruch 3, **dadurch gekennzeichnet, daß** die Hafniumverbindungen aus folgender Gruppe ausgewählt werden: HfOCl₂, HfCl₄, substituierte Hafnocen-Dichloride, substituierte Cyclopentadienyl-Hf-Trichloride, Hf-Acetylacetonat. Hf-Alkoxide, HfO₂, Hf(OH)₄, Hf(NO₃)₄, Hf(C₁₁H₁₉O₂)₄.

5. Verfahren nach einem der Ansprüche 1 bis 4, wobei der Katalysator einen Hafnium-Gehalt im Bereich von 0,01 bis 50 Gew.% aufweist.

6. Trägerkatalysator enthaltend auf einem porösem Trägermaterial als katalytisch aktive Komponenten Palladium sowie eine oder mehrere Alkaliverbindungen und eine oder mehrere Hafniumverbindungen erhältlich gemäß eines Verfahrens nach Anspruch 1.

7. Trägerkatalysator gemäß Anspruch 6, wobei der Katalysator einen Hafnium-Gehalt im Bereich von 0,01 bis 50 Gew.% aufweist

8. Trägerkatalysator gemäß Anspruch 6 der folgende Zusammensetzung aufweist:
PdₓAu_{y} Hf_{z},
wobei x= 0,7-1,3 Gew.%, y= 0,5-1 Gew.% und z= 0,05-5 Gew.% sind.

9. Trägerkatalysator nach einem der Ansprüche 6 bis 8 für die Herstellung von Vinylacetat.

## Claims

1. A process for producing a supported catalyst by impregnating the support with a basic solution and a solution comprising palladium salts, where the impregnation is carried out simultaneously or successively, with or without intermediate drying, washing the support to remove any chloride present and reducing the insoluble compounds precipitated on the support before or after washing, drying the catalyst precursor obtained in this way and impregnating it with alkali metal compounds which are converted completely or partially into alkali metal acetates under the reaction conditions in the production of vinyl acetate monomer, where the support is impregnated with one or more hafnium compounds either simultaneously with the noble metal impregnation or in an after-treatment and is dried at a temperature of ≤ 160°C and is not subsequently calcined.

2. The process as claimed in claim 1, wherein the support is impregnated with a solution which further comprises Au, Cd and/or Ba compounds as activators in addition to the palladium salts and the hafnium compound or compounds.

3. The process as claimed in claim 1 or 2; wherein hafnium compounds which are soluble in water, acetic acid or alcohol are used.

4. The process as claimed in claim 3, wherein the hafnium compounds are selected from the following group: HfOCl₂, HfCl₄, substituted hafnocene dichlorides, substituted cyclopentadienylhafnium trichlorides, hafnium acetylacetonate, hafnium alkoxides, HfO₂, Hf(OH)₄, Hf(NO₃)₄, Hf(C₁₁H₁₈O₂)₄).

5. The process as claimed in any one of claims 1 to 4, wherein the catalyst has a hafnium content in the range from 0.01 to 50% by weight.

6. A supported catalyst comprising palladium and one or more alkali metal compounds and one or more hafnium compounds as catalytically active components on a porous support material, and obtainable by a process as claimed in claim 1.

7. A supported catalyst as claimed in claim 6, wherein the catalyst has a hafnium content in the range from 0.01 to 50% by weight.

8. A supported catalyst as claimed in claim 6 having the following composition:
PdₓAu_{y}Hf_{z},
where x = 0.7-1.3% by weight, y = 0.5-1% by weight and z = 0.05-5% by weight.

9. A supported catalyst as claimed in any one of claims 6 to 8 for preparing vinyl acetate.

## Revendications

1. Procédé de production d'un catalyseur supporté pour la production d'acétate de vinyle par imprégnation du support avec une solution basique et une solution contenant des sels de palladium, où l'imprégnation se déroule simultanément ou successivement, avec ou sans séchage intermédiaire, lavage du support pour l'élimination des fractions de chlore éventuellement présentes et réduction des composés insolubles précipités sur le support avant ou après le lavage, séchage du processeur de catalyseur ainsi obtenu, et imprégnation avec des composés alcalins qui se transforment totalement ou partiellement en acétates alcalins dans les conditions réactionnelles lors de la production d'acétate de vinyle monomère où le support est imprégné simultanément avec l'imprégnation de métaux nobles ou dans un post-traitement avec un ou plusieurs composés du hafnium et est séché à une température ≤ 160°C après quoi il n'est pas calciné.

2. Procédé selon la revendication 1, **caractérisé en ce que** le support est imprégné d'une solution qui contient, outre les sels de palladium et le ou les composés de hafnium, des composés de Au, Cd et/ou Ba comme activateurs.

3. Procédé selon la revendication 1 ou 2, **caractérisé en ce que** l'on utilise des composés du hafnium solubles dans l'eau, l'acide acétique ou l'alcool.

4. Procédé selon la revendication 3, **caractérisé en ce que** les composés du hafnium sont choisis dans le groupe suivant : HfOCl₂, HfCl₄, dichlorures de hafnocène substitués, trichlorures de cyclopentadiényl-Hf substitués, acétylacétonate de Hf, alcoolates de Hf, HfO₂, Hf(OH)₄, Hf(NO₃)₄, Hf(C₁₁H₁₉O₂)₄.

5. Procédé selon l'une des revendications 1 à 4, où le catalyseur présente une teneur en hafnium dans le domaine de 0,01 à 50 % en masse.

6. Catalyseur supporté **caractérisé en ce qu'**il contient, sur un matériau support poreux, comme composants catalytiquement actifs du palladium ainsi qu'un ou plusieurs composés alcalins et un ou plusieurs composés du hafnium pouvant être obtenu par un procédé selon la revendication 1.

7. Catalyseur supporté selon la revendication 6, où le catalyseur présente une teneur en hafnium dans le domaine de 0,01 à 50 % en masse.

8. Catalyseur supporté selon la revendication 6, qui présente la composition suivante :
PdₓAu_{y}Hf_{z},
où x = 0,7-1,3 % en masse, y = 0,5-1 % en masse et z = 0,05-5 % en masse.

9. Catalyseur support selon l'une des revendications 6 à 8 pour la production d'acétate de vinyle.
